# EUROPEAN PATENT APPLICATION

(11) **EP 4 375 288 A1**
(43) Date of publication of application: **29.05.2024**
(21) Application number: 21946586.1
(22) Date of filing: 06.07.2021
(51) Int. Cl.: C07K 5/11, C07K 19/00, A61K 49/14, A61K 49/00, A61K 51/08, A61K 47/62, A61P 35/00

(54) **POLYPEPTIDE TARGETING INTEGRIN alpha6 AND USE THEREOF**

(30) Priority: 25.06.2021 CN 202110714315
(71) Applicant: Guangzhou Genbionova Medical Technology Co., Ltd., Guangzhou, Guangdong 510623 (CN)
(72) Inventor: ZENG, Musheng, Guangzhou, Guangdong 510060 (CN); FENG, Guokai, Guangzhou, Guangdong 510060 (CN); YE, Jiacong, Guangzhou, Guangdong 510060 (CN)
(74) Representative: Gassner, Birgitta
(86) International application number: PCT/CN2021/104639
(87) International publication number: WO 2022/267097

(57) **Abstract**

A polypeptide targeting integrin α6 and the use thereof. The polypeptide has a sequence motif of RWYD, and is stronger in affinity, wherein the equilibrium dissociation constant KD reaches the level of nM, and the affinity is greatly improved. The polypeptide has a strong affinity to integrin α6, a strong specificity and no biological toxicity, is relatively stable in vivo and reasonable in distribution, and can be used as a targeting carrier. In addition, the polypeptide targeting integrin α6 can be used as a molecular probe in the molecular imaging of various tumors with highly-expressed integrin α6. Further provided is a dimer of the polypeptide, which has a more significant targeting effect and no biological toxicity. The polypeptide targeting integrin α6 can be used in the molecular imaging of various tumors with highly-expressed integrin α6, and has an important application value in the molecular imaging of tumors.

## Description

### TECHNICAL FIELD

The present disclosure relates to the field of targeting peptide, and in particular, to a polypeptide targeting integrin α6 and use thereof.

### BACKGROUND

Integrin is a membrane protein with a large extracellular region, which is conducive to the proximity and binding of molecular probes, thus it is an ideal target for tumor molecular imaging and tumor targeted therapy. Integrins comprise 24 heterodimers formed by a combination of 18 types of α subunits and 8 types of β subunits. The integrins overexpressed in tumors mainly include αvβ3, αvβ5, α5β1, α4β1, α2β1, α3β1, αvβ6, α6β4 and α6β1. Among them, molecular probes of RGD polypeptide targeting integrin αvβ3 and tumor targeted therapeutic drugs are most widely studied. Integrin αvβ3 is overexpressed in tumor neovessels and underexpressed in blood vessels of normal tissue. Therefore, molecular probes of RGD polypeptide can be used to determine tumor neovascularization. So far, researchers have successfully developed a variety of radionuclide molecular probes, magnetic resonance molecular probes, ultrasonic molecular probes and optical molecular probes of RGD polypeptide. A considerable part of these molecular probes of RGD polypeptide have entered clinical trials and achieved good imaging effect in oncology patients.

Integrin α6 is present as a dimer of α6β4 and α6β1 in the body. Integrin α6 subunit is overexpressed in a variety of tumors, including breast cancer, liver cancer, lung cancer, colorectal cancer, esophageal cancer, glioma cancer, pancreatic cancer, prostate cancer, head and neck cancer, nasopharyngeal cancer, cervical cancer, gastric cancer, renal cancer, pheochromocytoma, and paraganglioma. Integrin α6 plays an important role in migration, invasion and proliferation of tumor cells. Meanwhile, GEPIA database showed that tumor patients with higher integrin α6 expression levels had worse overall survival rates and disease-free survival rates. Therefore, integrin α6 is an important marker for tumor diagnosis and prognostic evaluation. The development of polypeptide diagnostic agents targeting integrin α6 is of great significance for tumor diagnosis.

### SUMMARY

The objective of the present disclosure is to provide a novel polypeptide targeting integrin α6 and use thereof.

The technical scheme of the present disclosure is:
The first aspect of the present disclosure is to provide a polypeptide targeting integrin α6, comprising a motif of RWYD (SEQ ID NO: 1).

In some embodiments of the present disclosure, the amino acids include L-type amino acids and D-type amino acids.

The second aspect of the present disclosure is to provide a polymer of the polypeptide targeting integrin α6, which is polymerized from monomers of the polypeptide described in the first aspect of the present disclosure.

In some embodiments of the present disclosure, the polypeptide is connected with a linker.

In some preferred embodiments of the present disclosure, the linker is at least one of the following (I) to (II):
(I) a polyol;
(II) an amino acid.

In some embodiments of the present disclosure, the polyol is glycerol, pentaerythritol, xylitol, sorbitol or polyethylene glycol.

In some preferred embodiments of the present disclosure, the polyol is polyethylene glycol.

In some more preferred embodiments of the present disclosure, the polyol is a polyethylene glycol with a degree of polymerization of 3 to 12.

In some embodiments of the present disclosure, the number of the amino acids is 3 to 8.

In some embodiments of the present disclosure, the amino acid is any amino acid, preferably lysine.

In some embodiments of the present disclosure, the linker is attached to the C-terminus of the polypeptide.

In some preferred embodiments of the present disclosure, the polymer is a dimer.

The third aspect of the present disclosure provides is use of the polypeptide described in the first aspect of the present disclosure or the polymer described in the second aspect of the present disclosure in targeting agent of screening, diagnosis, treatment or prognostic assessment.

In some embodiments of the present disclosure, the targeting agent is a tumor targeting agent.

In some preferred embodiments of the present disclosure, the tumor is a tumor with high expression of integrin α6.

In some preferred embodiments of the present disclosure, the tumor is preferably liver cancer, breast cancer, lung cancer, colorectal cancer, esophageal cancer, glioma, pancreatic cancer, prostate cancer, head and neck cancer, nasopharyngeal cancer, cervical cancer, gastric cancer, renal cancer, pheochromocytoma, or paraganglioma.

In some embodiments of the present disclosure, the use may also be suitable for optical imaging, magnetic resonance imaging (MRI), positron emission imaging (PET), or single photon emission imaging (SPET) for navigation of NIRF area during clinical surgery.

The fourth aspect of the present disclosure is to provide a targeting agent comprising the polypeptide described in the first aspect of the present disclosure or the polymer described in the second aspect of the present disclosure.

In some embodiments of the present disclosure, the targeting agent further comprises an imaging agent and/or a therapeutic agent.

In some preferred embodiments of the present disclosure, the imaging agent is at least one of FB groups, radionuclides, biotins, fluorophores, fluorescent proteins, antibodies, horseradish peroxidase, and alkaline phosphatase.

In some more preferred embodiments of the present disclosure, the imaging agent is radionuclide.

In some embodiments of the present disclosure, the radionuclide labels the polypeptide described in the first aspect of the present disclosure or the polymer described in the second aspect of the present disclosure by a chelating agent.

In some preferred embodiments of the present disclosure, the radionuclide is ¹⁸F, ⁹⁹Tc, ⁶⁸Ga, ⁶⁴Cu, ¹¹¹In, or ¹⁷⁷Lu.

In some embodiments of the present disclosure, the chelating agent is HYNIC, DOTA, NOTA, or DTPA.

In some embodiments of the present disclosure, the therapeutic agent is at least one of radionuclides, proapoptotic peptides, nanoparticles, chemotherapy agents, nanodroplets, liposome drugs, and cytokines.

In some embodiments of the present disclosure, the targeting agent is a drug.

In some embodiments of the present disclosure, the dosage form of the drug is injection, granule, oral liquid, aerosol, capsule, or spray.

In some embodiments of the present disclosure, the drug is administered by intravenous, arterial, intracavitary, subcutaneous, or intrathecal injection.

In some embodiments of the present disclosure, the targeting agent is a tumor targeting agent.

In some preferred embodiments of the present disclosure, the tumor is a tumor with high expression of integrin α6.

In some more preferred embodiment of the present disclosure, the tumor is liver cancer, breast cancer, lung cancer, colorectal cancer, esophageal cancer, glioma, pancreatic cancer, prostate cancer, head and neck cancer, nasopharyngeal cancer, cervical cancer, stomach cancer, kidney cancer, pheochromocytoma, or paraganglioma.

The beneficial effects of present disclosure are as bellow.

The present disclosure provides a mutant RWYD, which has stronger affinity to integrin α6, the equilibrium dissociation constant KD of which reaches nM level, the affinity of which is greatly improved. It has strong affinity to integrin α6,a strong specificity, and no biological toxicity. It is relatively stable and reasonably distributed *in vivo,* and can be used as a targeting carrier. The polypeptide targeting Integrin α6 can be used as molecular probes in molecular imaging of a variety of tumors with high integrin α6 expression, such as optical imaging, magnetic resonance imaging (MRI), positron emission imaging (PET), or single photon emission imaging (SPET) and the like for navigation of NIRF area during clinical surgery, and has important application value in tumor molecular imaging.

The present disclosure also provides its dimer RD2, and it has also been fully verified that the dimer RD2 has a more significant targeting effect on tumors with high integrin α6 expression via cell flow binding assay, near-infrared imaging and PET/CT in mice. Based on REWD and RD2, the present disclosure also provides a targeting agent containing RWYD or RD2. It also contains imaging agent and/or therapeutic agent, and plays a better role in tumor diagnosis and treatment. The corresponding polypeptides labeled with imaging agent have better stability, and achieve better diagnostic effect.

### BRIEF DESCRIPTION OF THE DRAWINGS

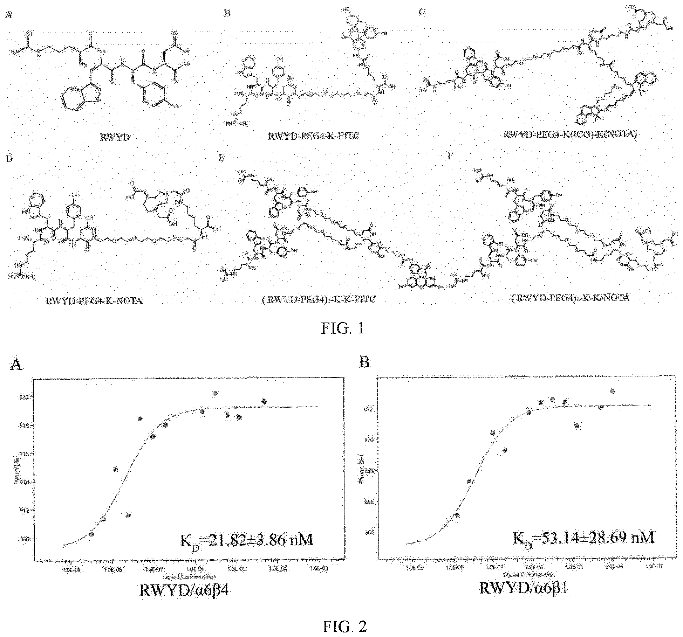

FIG. 1 depicts the structural formula of RWYD and its molecular imaging agent. Panel A shows a structural formula of RWYD; Panel B shows a structural formula of RWYD-PEG4-K-FITC; Panel C shows a structural formula of RWYD-PEG4-K(ICG)-K(NOTA); FIG. D shows a structural formula of RWYD-PEG4-K-NOTA; Panel E shows a structural formula of (RWYD-PEG4)2-K-K-FITC; Panel F shows a structural formula of (RWYD-PEG4)2-K-K-NOTA.
FIG. 2 depicts the strong affinity of RWYD to integrin α6β4 or α6β1. Panel A shows the affinity between RWYD and integrin α6β4; Panel B shows the affinity between RWYD and α6β1.

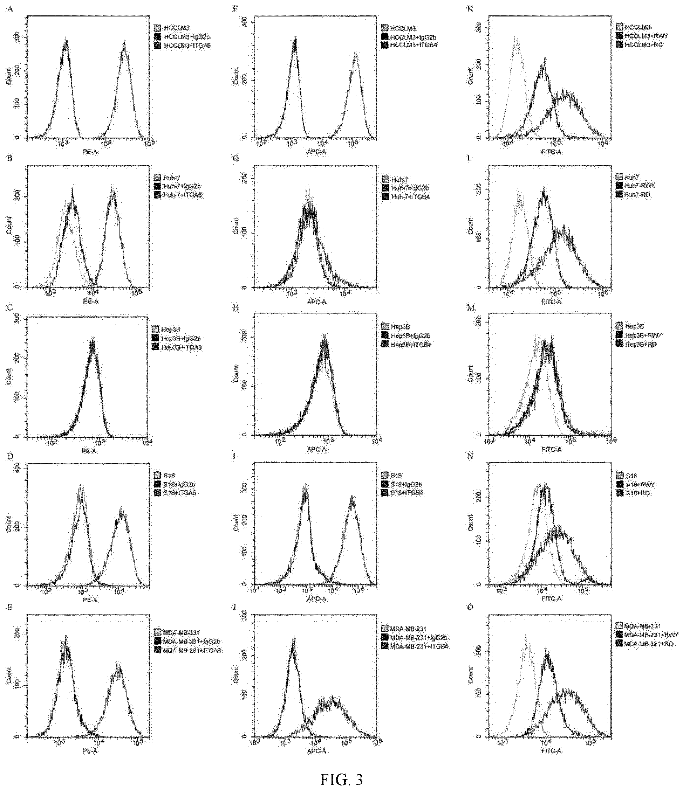

FIG. 3 depicts the strong affinity between RWYD and tumor cells with high integrin α6 expression; Panel A shows the expression of integrin α6 in HCCLM3 cells; Panel B shows the expression of integrin α6 in Huh-7 cells; Panel C shows the expression of integrin α6 in Hep3B cells; Panel D shows the expression of integrin α6 in S18 cells; Panel E shows the expression of integrin α6 in MDA-MB-231 cells; Panel F shows the expression of integrin β4 in HCCLM3 cells; Panel G shows the expression of integrin β4 in Huh-7 cells; Panel H shows the expression of integrin β4 in Hep3B cells; Panel I shows the expression of integrin β4 in S18 cells; Panel J shows the expression of integrin β4 in MDA-MB-231 cells; Panel K shows the affinity between RWY, RD and HCCLM3 cells with high integrin α6 expression; Panel L shows the affinity between RWY, RD and Huh7 cells with high integrin α6 expression; Panel M shows the affinity between RWY, RD and Hep3B cells; Panel N shows the affinity between RWY, RD and S18 cells with high integrin α6 expression. Panel O shows the affinity between RWY, RD and MDA-MB-231 cells with high integrin α6 expression.

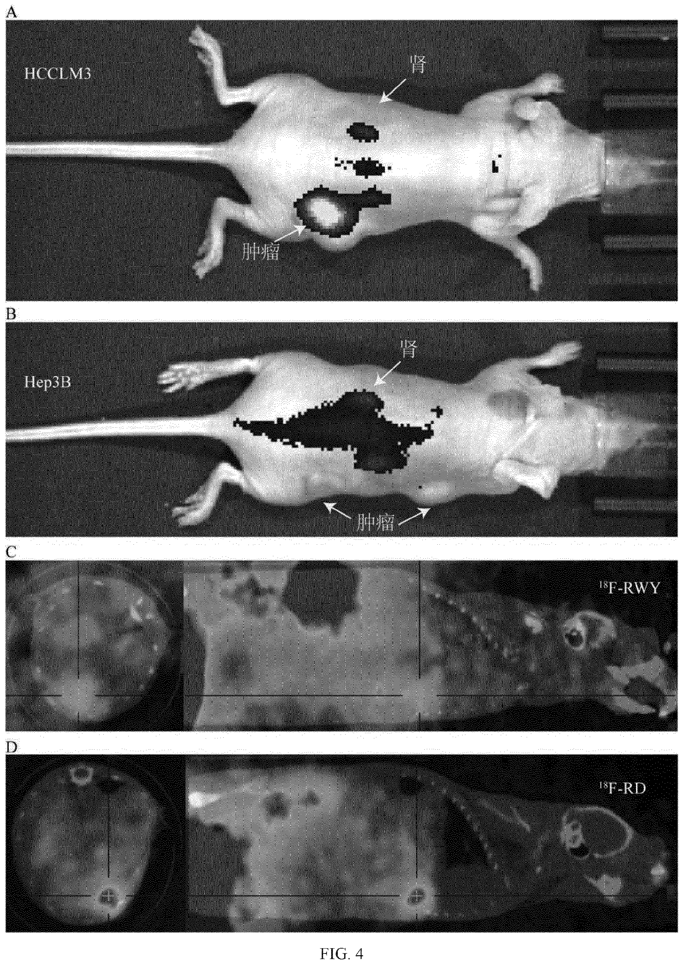

FIG. 4 depicts the *in vivo* targeting of RWYD to tumors with high integrin α6 expression. Panel A shows the high uptake of RWYD-PEG4-ICG by HCCLM3 subcutaneous tumors with high integrin α6 expression; Panel B shows that RWYD-PEG4-ICG is not high taken up by Hep3B subcutaneous tumors that do not express integrin α6; Panel C shows uptake by ¹⁸F-RWY labeling tumors; Panel D shows uptake by ¹⁸F-RD labeling tumors.

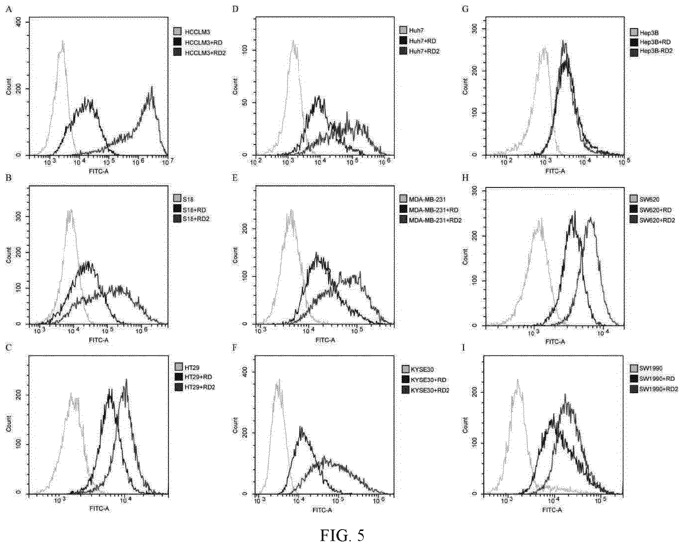

FIG. 5 depicts the strong affinity between RD, RD2 and tumor cells with high expression of integrin α6. Panel A shows the affinity between RD, RD2 and HCCLM3 cells; Panel B shows the affinity between RD, RD2 and S18 cells; Panel C shows the affinity between RD, RD2 and HT29 cells; Panel D shows the affinity between RD, RD2 and Huh7 cells; Panel E shows the affinity between RD, RD2 and MDA-MB-231 cells. Panel F shows the affinity between RD, RD2 and KYSE30 cells; Panel G shows the affinity between RD, RD2 and Hep3B cells; Panel H shows the affinity between RD, RD2 and SW620 cells; Panel I shows the affinity between RD, RD2 and SW1990 cells.

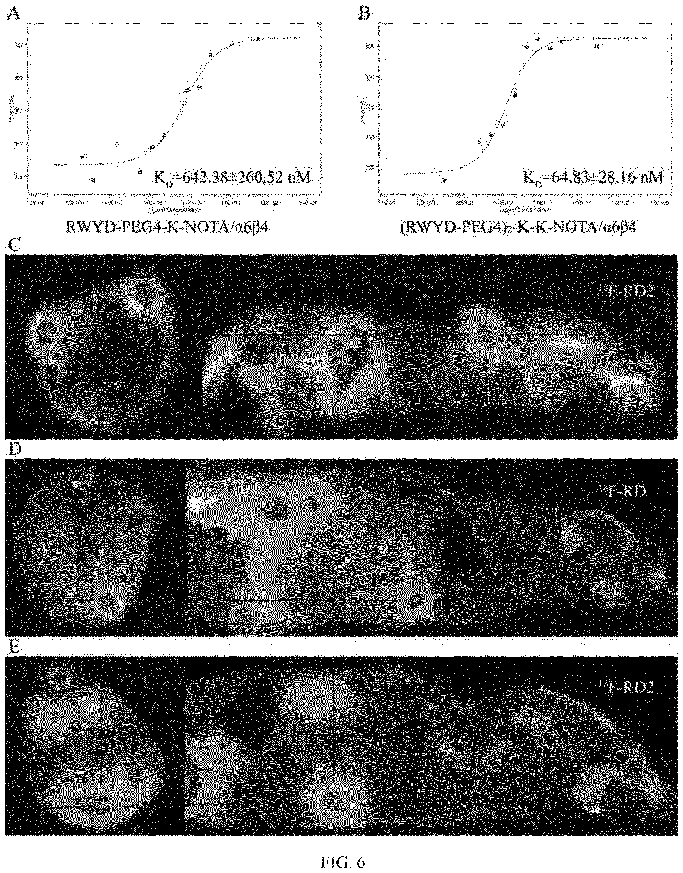

FIG. 6 depicts the *in vivo* targeting of RD2 to tumors with high integrin α6 expression. Panel A shows the affinity between RD and integrin α6β4; Panel B shows the affinity between RD2 and integrin a6β4; Panel C shows the uptake of ¹⁸F-RD2 at the tumor site in mice with subcutaneous HCCLM3 liver cancer; Panel D shows the uptake of ¹⁸F-RD at the tumor in mouse model of HCCLM3 in-situ liver cancer; Panel E shows the uptake of ¹⁸F-RD2 at the tumor site in a mouse model of HCCLM3 in-situ liver cancer.

### DETAILED DESCRIPTION

In the following, examples will be enumerated to clearly and completely illustrate the idea of the present disclosure and the resulting technical effects, in order to fully understand the purpose, features and effects of the present disclosure. Obviously, the examples described herein are only part of the examples of the present disclosure, not all of them. Based on the examples of the present disclosure, other examples obtained by person skilled in the art without creative labor are within the protection scope of the present disclosure.

### Example 1. Affinity assay of polypeptide mutant to integrin α6

As a further improvement of polypeptide targeting integrin α6, amino acid motifs are selected from the group consisting of: CRWYDENAC, CAWYDENAC, CRAYDENAC, CRWADENAC, CRWYAENAC, CRWYDANAC, CRWYDEAAC, RWYDENA, RWYDEN, RWYDANA, RWYDAN, RWYDEA, RWYDE, RWYDA, RWYD, RWYAEN, RWYAE and RWY Wherein CRWYDENAC, CAWYDENAC, CRAYDENAC, CRWADENAC, CRWYAENAC, CRWYDANAC, CRWYDEAAC are cyclic peptides formed by cysteine C at both ends. RWYDENA, RWYDEN, RWYDANA, RWYDAN, RWYDEA, RWYDE, RWYDA, RWYD, RWYAEN, RWYAE, and RWY are straight-chain peptides.

The affinity between these 18 polypeptides and integrin α6β4 was detected by microscale thermophoresis (MST) method as follows:
1) Integrin α6β4 protein was dissolved to 800nM, and polypeptide was dissolved to 100µM.
2) A RED-tris-NTA second-generation dye was diluted to 100nM by using the MO series RED-tris-NTA protein labeling kit.
3) 100 µL α6β4 protein was mixed with 50 µL dye and incubated at room temperature for 30min, so that the α6β4 protein binded to the dye.
4) 10 µL polypeptide was transferred into a PCR tube, and was diluted with PBST (containing 0.05% Tween 20) at a ratio of 1:1 to 16 gradients of concentrations .
5) 3 µL protein/dye mixture was transferred into a PCR tube, 3 µL of 16 different concentrations of polypeptides was respectively added and mixed well.
6) The mixture was absorbed by capillary and loaded on machine (instrument model Monolith NT.115) to assay, parameters were set as 80%LED/excitation power and medium MST power.
7) MO. Affinity Analysis software was used to calculate the equilibrium dissociation constant KD between α6β4 protein and polypeptide by KD fitting mode.

The results show that the linear quaternary polypeptide RWYD (structural formula shown in FIG. 1A) has the strongest affinity with integrin α6β4, and the affinity is much higher than that of the other 17 polypeptides. The KD is 21.82±3.86nM (FIG. 2A), which is much higher than that of CRWYDENAC (6.97±1.44µM). The affinity is increased for 327 times relative to that of CRWYDENAC (referred to as RWY) (Table 1). Furthermore, RWYD also has a strong affinity to integrin a6β1, with a K_{D} value of 53.14±28.69nM (FIG. 2B).

**Table 1 Affinity between polypeptide mutants and integrin α6β4**

| | Sequences | K_{D} (µm) | | | Mean value (µm) | Standard deviation | Affinity relative to RWY |
|---|---|---|---|---|---|---|---|
| | | 1 | 2 | 3 | | | |
| P1 | CRWYDENAC (SEQ IN NO: 2) | 8.59 | 6.52 | 5.81 | 6.97 | 1.44 | 1.00 |
| P2 | CAWYDENAC (SEQ IN NO: 3) | 4.03 | 12.13 | 3.58 | 6.58 | 4.81 | 1.06 |
| P3 | CRAYDENAC (SEQ IN NO: 4) | 14.40 | 11.76 | 13.85 | 13.34 | 1.39 | 0.52 |
| P4 | CRWADENAC (SEQ IN NO: 5) | | | | | | |
| P5 | CRWYAENAC (SEQ IN NO: 6) | 0.36 | 0.22 | 0.65 | 0.41 | 0.22 | 17.01 |
| P6 | CRWYDANAC (SEQ IN NO: 7) | 1.21 | 7.99 | 3.83 | 4.34 | 3.42 | 1.61 |
| P7 | CRWYDEAAC (SEQ IN NO: 8) | 0.25 | 0.25 | 1.37 | 0.62 | 0.65 | 11.19 |
| P8 | RWYDENA (SEQ IN NO: 9) | | | | | | |
| P9 | RWYDEN (SEQ IN NO: 10) | 9.69 | 8.73 | 13.09 | 10.50 | 2.29 | 0.66 |
| P10 | RWYDANA (SEQ IN NO: 11) | 62.05 | 17.53 | 12.38 | 30.65 | 27.31 | 0.23 |
| P11 | RWYDAN (SEQ IN NO: 12) | 34.65 | 23.19 | 35.69 | 31.18 | 6.94 | 0.22 |
| P12 | RWYDEA (SEQ IN NO: 13) | 5.26 | 2.49 | 1.74 | 3.16 | 1.85 | 2.20 |
| P13 | RWYDE (SEQ IN NO: 14) | 14.73 | 78.75 | 99.63 | 64.37 | 44.24 | 0.11 |
| P14 | RWYDA (SEQ IN NO: 15) | 6.28 | 4.69 | 4.22 | 5.06 | 1.08 | 1.38 |
| P15 | RWYD (SEQ IN NO: 1) | 0.020 | 0.018 | 0.026 | 0.02 | 0.00 | 326.875 |
| P16 | RWYAEN (SEQ IN NO: 16) | 7.31 | 6.08 | 9.74 | 7.71 | 1.86 | 0.90 |
| P17 | RWYAE (SEQ IN NO: 17) | | | | | | |
| P18 | RWY (SEQ IN NO: 18) | | | | | | |

### Example 2. Affinity assay of RWYD mutant (referred to as RD) to tumor cells with high integrin α6 expression

The expression of integrin α6 and β4 in human hepatoma cells HCCLM3, human hepatoma cells Huh-7, human hepatoma cells Hep3B, human nasopharyngeal carcinoma cells S18 and human breast cancer cells MDA-MB-231 were detected by flow cytometry. The results show that integrin α6 is expressed in HCCLM3, Huh-7, S18 and MDA-MB-231 cells (FIG. 3A, FIG. 3B, FIG. 3D, FIG. 3E). Integrin β4 is expressed in HCCLM3, S18 and MDA-MB-231 cells (FIG. 3F, FIG. 3G, FIG. 3I, FIG. 3J). Hep3B does not express α6 and β4 (FIG. 3C, FIG. 3H). The affinity between tumor cells and RWYD mutant was assayed by flow cytometry as follows:
1) Tumor cells were seeded into 12-well plates, culture medium was added and placed in the cell incubator for 24 hours, so that the cells would stick to the wall and grow. Each cell had 3 duplicate wells.
2) 2 µL of 1mM RWYD-PEG4-K-FITC (structural formula shown in FIG. 1B, referred to as RD) or CRWYDENAC-PEG4-K-FITC (referred to as RWY) was added into cells, mixed well, and incubated in an incubator at 37 °C for 2 hours.
3) The culture medium containing polypeptides was absorbed and removed, the cells were washed with PBS for 3 times, 500 µL/time.
4) The cells were digested with trypsin, resuspended with medium and centrifuged at 300g for 5 minutes.
5) The supernatant was discarded and cell precipitation was preserved, and the cells were resuspended with 500 µL saline and placed into the flow tube.
6) The FITC channel was detected by flow machine and the results were analyzed by CytExpert.

The results show that RD has a stronger affinity to tumor cells with high integrin α6 expression than RWY (FIG. 3K, FIG. 3L, FIG. 3N, FIG. 3O). The affinity of RD or RWY is consistent with Hep3B cells that does not express integrin α6 (FIG. 3M).

### Example 3. In vivo targeting assay of RD to tumors with high integrin α6 expression

In the near infrared imaging of small animals, a 2µL of 1mM fluorescent probe RWYD-PEG4-K(ICG)-K(NOTA) (structural formula shown in FIG. 1C) was injected into the tail vein of HCCLM3 subcutaneous tumor bearing mice. After 24 hours of internal circulation, the distribution in the subcutaneous tumor bearing mice was observed. Results show that RWYD-PEG4-K(ICG)-K(NOTA) is high taken up by HCCLM3 subcutaneous tumors with high integrin α6 expression (FIG. 4A), but is not high taken up by Hep3B subcutaneous tumors without expression of integrin α6 (FIG. 4B).

Tumor targeting of RD in HCCLM3 in-situ hepatocellular carcinoma mice was detected by PET/CT in small animals. The specific operations were as follows:
1) 100 µg RWYD-PEG4-K-NOTA (structural formula shown in FIG. 1D), 6.275 µg AlCl₃·6H₂O, 330 µL anhydrous ethanol or acetonitrile, 5µL acetic acid, 65µL 18F ionized water with an activity of about 3 mCi, were mixed to obtain a mixture with a pH of approximately 4, and then placed the mixture in a 10 mL vacuum glass bottle.
2) The vacuum glass bottle containing the mixture was heat in a metal bath at 100°C for 10 minutes, and then leave it at room temperature for 5 minutes.
3) Waters Sep-Pak C18 column was activated, washed with 10 mL anhydrous ethanol, and then washed with 20 mL sterilized water.
4) 10 mL sterilized water was added into the glass bottle, mixed well, transferred to the activated C18 column, washed with 20 mL sterilized water, and eluted with 400 µL anhydrous ethanol.
5) Anhydrous ethanol was evaporated by termovap sample concentrator, and 300 µL saline was added for dissolution.
6) 10µCi was tested by HPLC for quality control.
7) Approximately 100µCi was taken and injected into mice through the tail vein.
8) After 1 hour, the mice were anesthetized by isoflurane and placed in the testing chamber for PET/CT imaging.

The results show that the tumor uptake of ¹⁸F-labeled RWYD-PEG4-K-NOTA (referred to as ¹⁸F-RD) is clearly visible and significantly better than that of ¹⁸F-labeled CRWYDENAC-PEG4-K-NOTA (referred to as ¹⁸F-RWY), except for a small amount of uptake by kidney and bladder, background of other organs is lower (FIG. 4C, FIG. 4D). Both the near infrared imaging and PET/CT results in small animals *in vivo* demonstrates that RD mutant has stronger integrin α6 targeting and specificity than RWY

### Example 4. Affinity assay of RWYD dimer (referred to as RD2) to tumor cells with high integrin α6 expression

RWYD-PEG4 forms dimers or polyploids via lysine (K) ligations. Flow cytometry was used to detect expression of integrin α6 in tumor cells. Results show that all of HCCLM3, Huh-7, S18, MDA-MB-231, human colorectal adenocarcinoma cell SW620, human colorectal adenocarcinoma cell HT29, human esophageal squamous cell KYSE30 and human pancreatic cancer cell SW1990 express high levels of integrin α6,while Hep3B does not. The specific operation of affinity assay of tumor cells to RD2 is similar to that of "affinity assay of tumor cells to RD". The results show that compared with RWYD-PEG4-K-FITC, (RWYD-PEG4)₂-K-K-FITC (structural formula shown in FIG. 1E) has stronger affinity to tumor cells with high integrin α6 expression (FIG. 5A-FIG. 5F, FIG. 5H-Fig. 5I), whereas RD2 or RD has similar affinity to Hep3B cells that does not express integrin α6 (FIG. 5G). These results demonstrate that the affinity of RD to integrin α6 may be increased by constructing RD2 dimer or polyploid at cellular level.

### Example 5. In vivo targeting of RD2 dimer to tumors with high expression of integrin α6

By MST method, it is determined that (RWYD-PEG4)₂-K-K-NOTA (structural formula shown in FIG. 1F) has a stronger affinity to integrin α6β4, and the KD is 64.83±28.16nM (FIG. 6B), which is much larger than the 642.38±260.52nM of RWYD-PEG4-K-NOTA (FIG. 6A), and the affinity is increased by about 10 times. Tumor targeting of RD2 in HCCLM3 subcutaneous hepatocellular carcinoma mice and in situ hepatocellular carcinoma mice was detected by PET/CT in small animals. The specific operation was similar to that of "detection of tumor targeting of RD in HCCLM3 in-situ hepatocellular carcinoma mice". In HCCLM3 mice with subcutaneous liver cancer, uptake of ¹⁸F-labeled (RWYD-PEG4)₂-K-K-NOTA (referred to as ¹⁸F-RD2) is clearly visible at tumor site (FIG. 6C). In the mouse model of HCCLM3 in-situ liver cancer, the uptake effect of ¹⁸F-RD2 at the tumor site is significantly better than that of ¹⁸F-RD, except for a small amount of uptake by kidney and bladder, background of other organs is lower (FIG. 6D, FIG. 6E). These results demonstrate that, in tumor-bearing mouse model, RD2 dimer has a strong affinity to integrin α6 and a strong molecular targeting effect in tumors with high expression of integrin α6.

The above-mentioned specific embodiments are described in detail for the present disclosure, but the present disclosure is not limited to the embodiments and may be altered within the scope of knowledge of ordinary technicians in the technical field without deviating from the purpose of the present disclosure. In addition, embodiments of the present disclosure and features in the embodiments may be combined with each other without conflict.

## Claims

1. A polypeptide targeting integrin α6, comprising a motif of RWYD.

2. A polymer of the polypeptide targeting integrin α6, wherein the polymer is polymerized from monomers of the polypeptide according to claim 1.

3. The polymer according to claim 2, wherein the polypeptide is connected with a linker, preferably the linker is at least one of the following (I) to (II) :
(I) a polyol;
preferably, the polyol is glycerol, pentaerythritol, xylitol, sorbitol, polyethylene glycol;
more preferably, the polyethylene glycol is a polyethylene glycol with a degree of polymerization of 3 to 12;
(II) an amino acid;
preferably, the number of the amino acids is 3 to 8;
more preferably, the amino acid is lysine.

4. The polymer according to any one of claims 2 to 3, wherein the polymer is a dimer.

5. Use of the polypeptide according to claim 1 or the polymer according to any one of claims 2 to 4 in the preparation of targeting agents of screening, diagnosis, treatment or prognostic assessment.

6. A targeting agent comprising the polypeptide according to claim 1 or the polymer according to any one of claims 2 to 4.

7. The agent according to claim 6, wherein the agent further comprises an imaging agent and/or a therapeutic agent.

8. The agent according to claim 7, wherein the imaging agent is at least one of FB groups, radionuclides, biotins, fluorophores, fluorescent proteins, antibodies, horseradish peroxidase, and alkaline phosphatase; preferably, the therapeutic agent is at least one of radionuclides, proapoptotic peptides, nanoparticles, chemotherapy agents, nanodroplets, liposome drugs, and cytokines.

9. The agent according to claim 8, wherein the nuclide labels the polypeptide according to claim 1 or the polymer according to any one of claims 2 to 4 by a chelating agent; preferably, the radionuclide is ¹⁸F, ⁹⁹Tc, ⁶⁸Ga, ⁶⁴Cu, ¹¹¹In, or ¹⁷⁷Lu; more preferably, the chelating agent is HYNIC, DOTA, NOTA, or DTPA.

10. The agent according to any one of claims 6 to 9, wherein the targeting agent is a tumor targeting agent, the tumor is a tumor with high expression of integrin α6; preferably, the tumor is liver cancer, breast cancer, lung cancer, colorectal cancer, esophageal cancer, glioma, pancreatic cancer, prostate cancer, head and neck cancer, nasopharyngeal cancer, cervical cancer, stomach cancer, kidney cancer, pheochromocytoma, or paraganglioma.
